# EUROPÄISCHE PATENTSCHRIFT

(11) **EP 1 239 963 B1**
(45) Veröffentlichungstag und Bekanntmachung des Hinweises auf die Patenterteilung: **07.05.2003**
(21) Anmeldenummer: 00993589.1
(22) Anmeldetag: 22.12.2000
(51) Int. Cl.: B01L 3/14, A61B 5/15

(54) **VERSCHLUSSVORRICHTUNG FÜR EINEN UNTERDRUCK-PROBENSAMMELBEHÄLTER**
CLOSURE DEVICE FOR A VACUUM SAMPLE COLLECTOR
DISPOSITIF DE FERMETURE POUR UN COLLECTEUR D'ECHANTILLONS SOUS VIDE

(30) Priorität: 23.12.1999 DE 19962664
(43) Veröffentlichungstag der Anmeldung: 18.09.2002
(73) Patentinhaber: Helvoet Pharma Belgium N.V., 3570 Alken (BE)
(72) Erfinder: CLAESSENS, Albert, Louis, Victor, Jozef, B-3530 Houthalen (BE)
(74) Vertreter: Fehners, Klaus Friedrich, Dipl.-Ing., Dipl.-Wirtsch.-Ing.
(86) Internationale Anmeldenummer: EP0013169
(87) Internationale Veröffentlichungsnummer: WO01047636

(56) Entgegenhaltungen:
- US-A- 5 361 921
- US-A- 5 494 170
- US-A- 5 779 074
- PATENT ABSTRACTS OF JAPAN vol. 2000, no. 02, 29. Februar 2000 (2000-02-29) -& JP 11 318868 A (SEKISUI CHEM CO LTD), 24. November 1999 (1999-11-24)

## Beschreibung

Die Erfindung bezieht sich auf eine Verschlußvorrichtung für einen Unterdruck-Probensammelbehälter, umfassend eine Außenkappe aus einem harten Material, vorzugsweise aus einer Kunststoff-Hartkomponente, mit einer Stirnwand und einer diese umgebenden Seitenschürze, die auf einen hülsenartigen Öffnungsabschnitt des Unterdruck-Probensammelbehälters aufsetzbar ist, und ein in der Außenkappe behälterseitig angebrachtes Dichtelement aus einem elastischen Dichtmaterial, vorzugsweise einer Kunststoff-Weichkomponente, wobei die Stirnwand der Außenkappe nach innen und eine Vertiefung bildend eingestülpt ist und der Boden dieser Einstülpung wiederum nach außen gerichtet umgestülpt ist, und diese Umstülpung als ein im wesentlichen hohlzylindrischer Vorsprung ausgebildet ist, der einen glattflächigen Durchgang aufweist, dessen Öffnung in einem Abstand von einem Stirnende der Außenkappe mündet, der außerhalb eines Eingriffsbereichs eines Fingers liegt, und daß zwischen der Außenwand des zylindrischen Vorsprungs und der Innenwand der nach innen gerichteten Einstülpung ein enger, spaltförmiger Ringraum gebildet ist, wobei das Dichtelement den Durchgang ausfüllt und weiterhin die Innenwand der Einstülpung sowie eine Hohlkehle, die an dem Stirnende der Außenkappe innenseltig zwischen der Seitenschürze und der Einstülpung gebildet ist, auskleidet, wobei zu der Innenwand der Seitenschürze ein Spalt zur dichtenden Aufnahme des hülsenartigen Öffnungsabschnittes des Unterdruck-Probensammelbehälters ausgebildet ist.

Derartige Verschlußvorrichtungen werden vor allem für Blutprobenröhrchen bei der Blutabnahme verwendet. Die zunächst leeren, einen Unterdruck aufweisenden und mit einer Verschlußvorrichtung abgedichteten Blutprobenröhrchen werden bei der Blutabnahme im Bereich der Verschlußvorrichtung mit einem ersten Ende einer doppelendigen Kanüle angestochen, die mit einem zweiten Ende in eine Vene einer Person eingeführt ist. Durch den in dem Blutprobenröhrchen vorherrschenden Unterdruck wird das Blut in das Blutprobenröhrchen gesogen. Nach der Befüllung des Röhrchens wird dieses zusammen mit der Verschlußvorrichtung von der Kanüle abgezogen, wobei die Verschlußvorrichtung eine automatische Abdichtung des Blutprobenröhrchens bewirkt. An die gegebenenfalls noch an der Person befindliche Kanüle kann ein weiteres Blutprobenröhrchen mit einer Verschlußvorrichtung zur Abnahme einer weiteren Blutmenge angekoppelt werden.

In einem Untersuchungslabor wird die Verschlußvorrichtung von dem Blutprobenröhrchen entfernt, so daß das Blut analysiert werden kann. Eine andere Möglichkeit besteht darin, durch die Verschlußkappe einzustechen und eine Probe mit einer Kanüle abzuziehen.

Die Verschlußvorrichtung kann auch zu anderen Zwecken eingesetzt werden, bei denen eine Abdichtung eines Probensammelbehälters mit einem gegenüber der Umgebung verringerten Innendruck benötigt wird. Der in dem Probensammelbehälter befindliche Innendruck wird während eines Entnahmevorganges einer Probe zum Ansaugen einer Flüssigkeit zur Wirkung gebracht, indem die Abdichtung des Probensammelbehälters zur Umgebung im Bereich der Verschlußvorrichtung zeitweilig mit einer hohlen Nadel aufgehoben wird.

In allen Anwendungsfällen ist es erforderlich, daß die Verschlußvorrichtung an dem Unterdruck-Probensammelbehälter dicht abschließt, um den Unterdruck bis zum Einsatzzeitpunkt aufrechtzuerhalten und eine Kontamination des Behälterinhaltes mit Fremdstoffen zu vermeiden, die das Analyseergebnis verfälschen würden.

Weiterhin soll das Durchstechen der Verschlußvorrichtung mittels einer Kanüle mit möglichst geringem Widerstand erfolgen. Dies ist insbesondere dann wünschenswert, wenn die Verschlußvorrichtung im Zusammenhang mit einem Blutprobenröhrchen verwendet wird, da bei der Blutabnahme meist mehrere Röhrchen befüllt werden, wobei die Kanüle an der Person eingestochen bleibt. Stärkere Widerstandkräfte beim Durchstechen der Verschlußvorrichtung können sich auf Seiten der Person schmerzhaft bemerkbar machen.

Trotz einer geringen Einstechkraft soll die Kanüle jedoch während einer Blutabnahme in der Verschlußvorrichtung sicher gehalten werden. Der Verminderung des Einstechwiderstandes steht daher das zusätzliche Erfordernis einer ausreichenden Fixierung der Kanüle während eines Entnahmevorganges entgegen.

Bei der Blutentnahme bleibt manchmal während des Abziehens des Blutprobenröhrchens von der Kanüle an der Spitze der Kanüle ein Bluttropfen hängen, der sich an der Einstechstelle der Verschlußvorrichtung verteilt. Befindet sich die Einstechstelle im Griffbereich der Finger einer das Blutprobenröhrchen handhabenden Person, so besteht für diese ein erhöhtes Infektionsrisiko. Zudem kann es beim Herausziehen der Kanüle aus der Verschlußvorrichtung zu Spritzeffekten kommen. Diese treten beispielsweise dann auf, wenn das Material um die Einstechstelle ein niedriges Formrückstellvermögen aufweist und nach einem Herausziehen der Kanüle aus der Verschlußvorrichtung sich nicht schnell genug wieder verschließt. Auch beim Öffnen der Verschlußvorrichtung im Untersuchungslabor ist sicherzustellen, daß bei einer Aufhebung des noch in dem Blutprobenröhrchen verbleibenden Restunterdrucks keine Spritzeffekte nach außen hin wirksam werden.

Überdies ist zu beachten, daß aus Gründen der Sterilität die Verschlußvorrichtungen nur einmal verwendet werden dürfen, so daß die Verschlußvorrichtungen in hoher Anzahl benötigt werden. Fertigungstechnische sowie materialspezifische Gesichtspunkte können somit für den Produkterfolg entscheidend sein.

Aus dem Stand der Technik sind eine Vielzahl von Verschlußvorrichtungen bekannt, die größtenteils in einem einzigen Stück aus einem elastischen Kunststoff oder Gummi hergestellt sind. Ein Beispiel hierfür ist in der US 3,974,930 A angegeben. Da diese bekannte Verschlußvorrichtung neben einem zu durchstechenden Abschnitt gleichzeitig der Abschirmung des Unterdruckes dient, muß diese großvolumig aus einem Material mit verhältnismäßig hohem Formrückstellvermögen ausgebildet werden, damit nicht durch ein versehentlich zu festes Angreifen an der Verschlußvorrichtung der Unterdruck in dem Probensammelbehälter abgebaut wird. Die Materialauswahl bedingt eine hohe Widerstandskraft beim Durchstechen mit einer Kanüle. Um die Einstechstelle gegen die Berührung durch die Finger einer handhabenden Person abzuschirmen, ist ein enger Mittelkanal vorgesehen, in den die Spitze einer Kanüle nur schwer einzuführen ist und der überdies zu einer erheblichen Wanddicke und damit zu einem hohen Materialbedarf führt.

Eine weitere Verschlußeinrichtung ist aus der US 5,494,170 A bekannt, bei der ein Dichtelement aus einem Elastomer in eine Außenkappe aus hartem Kunststoff eingesetzt ist. Das Dichtelement erstreckt sich hierbei durch eine in der Außenkappe vorgesehene Öffnung und deckt einen Boden einer in der Außenkappe stirnseitig vorgesehenen Vertiefung breitflächig ab, so daß der zu durchstechende Bereich von außen visuell nicht exakt erkennbar ist. Im zu durchstechenden Bereich ist an dem Dichtelement eine kegelstumpfartige Erhebung vorgesehen, die das Ablaufen eines an der Kanülenspitze manchmal anhängenden Tropfens in die Breitfläche um die Erhebung herum begünstigt. Dabei liegen sowohl die Erhebung als auch die Breitfläche außerhalb eines Bereiches eines an der Stirnseite der Außenkappe flach anliegenden Fingers. Aufgrund des großen Durchmessers der Vertiefung kann jedoch nicht gewährleistet werden, daß bei einer insachgemäßen Handhabung eine Fingerspitze in Anlage gegen die Erhebung oder die Breitfläche und dort in Berührung mit einem Bluttropfen gelangt. Überdies ist der Materialeinsatz in bezug auf die teurere Weichkomponente nicht optimal. Die membranartige Ausbildung der Außenkappe im Bereich um die Einstechstelle bedingt entweder eine hohe Wanddicke oder aber eine nicht befriedigende Steifigkeit der Außenkappe im Bodenbereich der Vertiefung.

Eine Verschlußvorrichtung, die der eingangs dargestellten Verschlußvorrichtung nahekommt, ist aus der US 5,779,074 A bekannt, bei der allerdings der zylindrische Vorsprung bis zur Oberkante der Stirnwand hochgezogen ist, um das Eindringen eines Fingers in die Umstülpung zu vermeiden. Des weiteren weist die Öffnung auch einen verhältnismäßig großen Durchmesser auf, der letztlich nicht mit großer Sicherheit das Eindringen einer Fingerkuppe in diesen Durchstichbereich verhindert. Insbesondere ist aber festzustellen, daß beim Herausziehen einer Kanüle aus dem in der Öffnung vorgesehenen Dichtelement ein dabei abgestreifter Bluttropfen nicht in den spaltförmigen Ringraum ablaufen und dort haften bleiben kann.

Der Erfindung liegt demgegenüber die Aufgabe zugrunde, eine Verschlußvorrichtung der eingangs genannten Art zu schaffen, die bei Gewährleistung einer hohen Dichtwirkung, einer geringen Infektionsgefahr und einem geringen Einstechwiderstand mit geringem Materialeinsatz einfach herstellbar ist.

Diese Aufgabe wird bei der eingangs beschriebenen Verschlußvorrichtung dadurch gelöst, daß die Höhe des zylindrischen Vorsprungs über dem Boden der Einstülpung etwa die Hälfte der Tiefe der Einstülpung beträgt und daß die Höhe der Seitenschürze in etwa das Doppelte der Tiefe der Einstülpung beträgt und daß an der Innenwand der nach innen gerichteten Einstülpung in die Vertiefung hineinragende, radiale Versteifungsrippen ausgebildet sind.

Diese erfindungsgemäße Lösung gewährleistet, daß der Vorsprung bzw. die Öffnung dem Griffbereich einer Fingerkuppe mit Sicherneit entzogen ist, so daß das Infektionsrisiko für die handhabende Person ausgeschaltet ist. Die radialen Versteifungsrippen verringern ohne größeren Materialaufwand den freien Durchmesser der Vertiefung, so daß selbst eine schmale Fingerkuppe nicht in den Infektionsbereich an den Vorsprung gelangen kann. Des weiteren wird durch die Versteifungsrippen in Verbindung mit dem Ringspalt ein Raum geschaffen, in dem abspritzende Bluttropfen sicher aufgefangen werden.

Aufgrund der engen, spaltförmigen Ausbildung des Ringraumes wird eine breitflächige Verteilung des Tropfens vermieden, so daß dessen Gesamtoberfläche gering bleibt. Zudem tritt durch die spaltförmige Ausbildung ein gewisser Sogeffekt auf, der den Tropfen an die tiefste Stelle des Ringraumes zieht.

Darüber hinaus ermöglicht die erfindungsgemäße Lösung auch ein sicheres Abdichten des Unterdruck-Probensammelbehälters, das auch durch eine seitliche Belastung der Außenkappe nicht beeinträchtigt wird, da der hülsenartige Öffnungsabschnitt gegen die feste Innenwand der Außenkappe gepreßt wird. Das Dichtelement dient hierbei als zusätzliche Druckfeder, welche die Anlagekraft des hülsenartigen Öffnungsabschnittes gegen die Innenwand der Seitenschürze der Außenkappe unterstützt.

Durch die bodenseitig in der Vertiefung der Außenkappe vorgesehene Umstülpung, d. h. den im wesentlichen zylindrischen Vorsprung, wird im Bereich der Einstechstelle der Verschlußkappe ein Versteifungseffekt bewirkt, der eine besonders dünnwandige Ausführung der Außenkappe in diesem Bereich ermöglicht, wobei gleichzeitig eine hohe strukturelle Festigkeit erreicht wird. Zudem kann die Menge des benötigten Dichtmaterials auf ein Minimum reduziert werden. Da das Dichtmaterial überdies nicht zur Versteifung des Bodenbereichs der Vertiefung benötigt wird, kann dieses allein im Hinblick auf einen möglichst geringen Widerstand beim Durchstechen ausgewählt werden. Hierdurch ergibt sich eine Verschlußkappe, die bei Verwendung im Zusammenhang mit einem Blutprobenröhrchen eine für eine Person weniger belastende Blutabnahme erlaubt, da der Wechsel der Röhrchen mit geringen Reaktionskräften ausgeführt werden kann.

In einer vorteilhaften Ausgestaltung erstrecken sich die radialen Versteifungsrippen bis in den spaltförmigen Ringraum und unterteilen diesen in spaltförmige Taschen. Damit ist eine weitere Erhöhung der strukturellen Steifigkeit des Bodenbereiches der Außenkappe verbunden, die weitere Materialeinsparungen erlaubt.

Zur Optimierung der Festigkeit sowie für des bereits erwähnten Sogeffektes ist es vorteilhaft, wenn die maximale Breite des engen, spaltförmigen Ringraums das 1,5-fache der Wanddicke der Einstülpung an der Außenkappe nicht überschreitet.

Eine weitere Optimierung des Materialeinsatzes des elastischen Dichtmaterials ergibt sich bevorzugt dann, wenn das Dichtelement sich bis zu einem Innenrand der Mündungsöffnung des zylindrischen Vorsprungs erstreckt und in dieser endet. Darüber hinaus wird dadurch auch noch das Ablaufen eines Tropfens in den Ringspalt ermöglicht und auch noch dadurch verbessert, daß in einer weiteren, bevorzugten Ausgestaltung der Erfindung das durch die Mündungsöffnung und das Dichtelement gebildete Ende des zylindrischen Vorsprungs nach außen ballig ausgebildet ist.

In einer weiteren, vorteilhaften Ausgestaltungsform ist die Verschlußvorrichtung als Zweikomponenten-Kunststoffspritzgußteil ausgebildet, wobei sich zwischen der Außenkappe und dem Dichtelement eine Verbundhaftung und damit eine hohe Verbindungskraft einstellt. Vorzugsweise ist an dem Stirnende der Außenkappe im Übergang der Seitenschürze zu der Einstülpung mindestens eine Durchgangsöffnung ausgebildet, die von außen zu der Hohlkehle leitet. Hierdurch läßt sich bei einer spritzgußtechnischen Herstellung ein gutes Füllungsverhalten beim Spritzen des Dichtelementes erzielen, so daß der Ausschuß in der Fertigung gering bleibt.

In einer weiteren, bevorzugten Ausgestaltung der Erfindung ist die Außenkappe aus einem gasundurchlässigen Material, vorzugsweise Polypropylen und das Dichtelement aus einem Elastomer, vorzugsweise Gummi hergestellt. Eine solche Verschlußvorrichtung weist eine erheblich größere Gasdichtigkeit auf, als eine allein aus Gummi hergestellte Kappe.

Im Sinne der Erfindung wird weiterhin ein Unterdruck-Probensammelbehälter, vorzugsweise zur Verwendung bei der Blutabnahme, geschaffen, der einen Behälter mit einem hülsenartigen Öffnungsabschnitt umfaßt, der durch eine Verschlußvorrichtung der vorstehend beschriebenen Art abgedichtet ist, wobei in dem Behälter ein Unterdruck vorherrscht. Hierdurch ergibt sich ein Probenbehälter, der mit einer hohen Dichtwirkung ausgestattet ist, und der bei einer geringen Infektionsgefahr und einem geringen Einstechwiderstand mit geringem Materialeinsatz einfach herstellbar ist.

Die Erfindung wird nachfolgend anhand eines in der Zeichnung dargestellten Ausführungsbeispiels näher erläutert. Die Zeichnung zeigt in
- Figur 1: eine räumliche Darstellung des Ausführungsbeispiels einer Verschlußvorrichtung,
- Figur 2: eine weitere räumliche Darstellung des Ausführungsbeispiels, bei der aus Gründen der Veranschaulichung ein Viertel-Segment weggeschnitten ist,
- Figur 3: einen Vertikalschnitt durch eine Außenkappe der Verschlußvorrichtung,
- Figur 4: einen Vertikalschnitt durch ein Dichtelement der Verschlußvorrichtung,
- Figur 5: eine Ansicht von oben auf die Verschlußvorrichtung,
- Figur 6: einen Vertikalschnitt durch die Außenkappe und das Dichtelement im zusammengebauten Zustand,
- Figur 7: eine räumliche Darstellung eines Unterdruck-Probensammelbehälters in Form eines Blutprobenröhrchens mit einer angesetzten Verschlußvorrichtung,
- Figur 8: eine räumliche Darstellung des Blutprobenröhrchens mit der angesetzten Verschlußvorrichtung, bei der aus Gründen der Veranschaulichung an der Verschlußvorrichtung ein Viertel-Segment weggeschnitten ist
- Figur 9: die Verwendung des Blutprobenröhrchens im Zusammenhang mit einer doppelendigen Kanüle, die in etwa mittig an einem hülsenartigen Halter befestigt ist, wobei der Zustand vor dem Anstechen der Verschlußvorrichtung gezeigt ist, und in
- Figur 10: eine räumliche Darstellung entsprechend Figur 9, jedoch mit der Verschlußvorrichtung im angestochenen Zustand.

Das in den Figuren dargestellte Ausführungsbeispiel zeigt eine Verschlußvorrichtung 1, die im folgenden im Zusammenhang mit einem Unterdruck-Probensammelbehälter 30 in Form eines Blutprobenröhrchens erläutert wird.

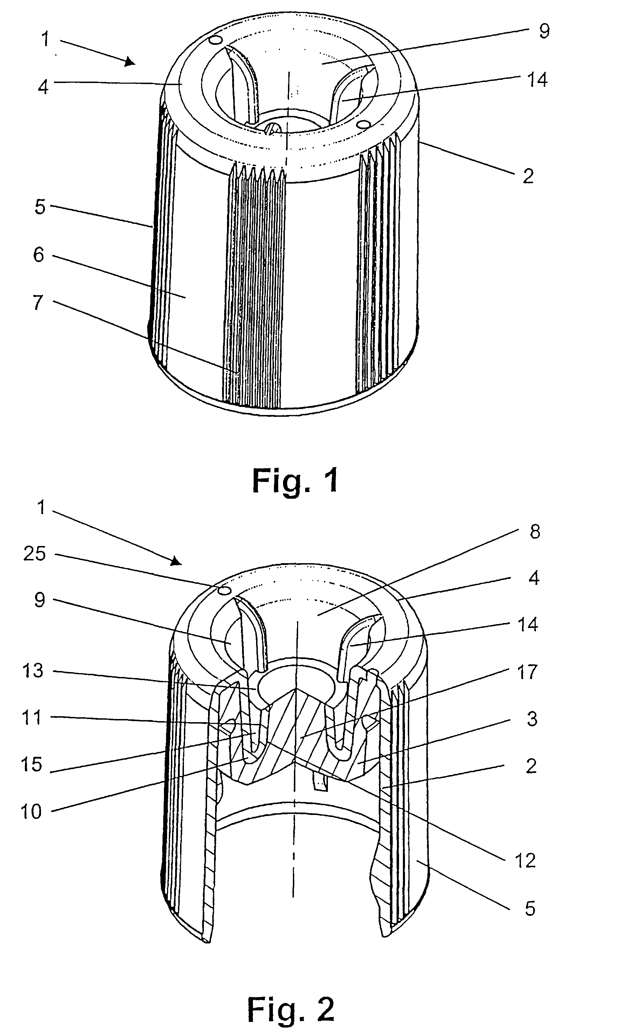

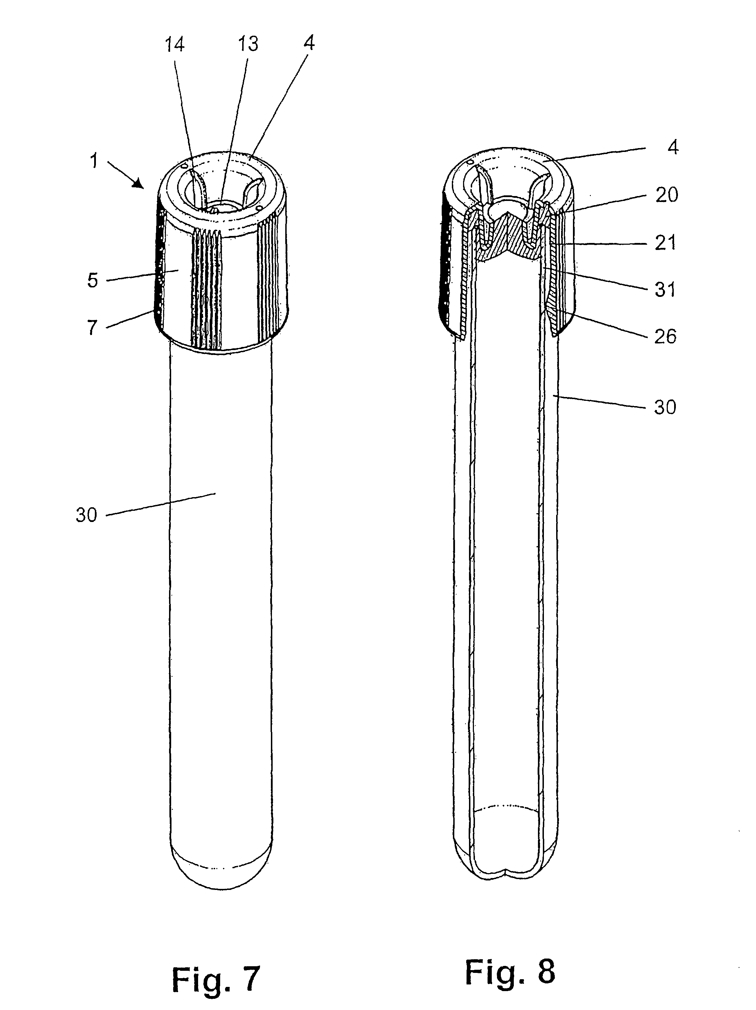

Figur 2 zeigt die Verschlußvorrichtung 1, die aus einer Außenkappe 2 und einem Dichtelement 3 gebildet ist. Die Verschlußvorrichtung 1 wird, wie in Figur 8 gezeigt, auf einen hülsenartigen Öffnungsabschnitt 31 eines Unterdruck-Probensammelbehälters 30 aufgesetzt, um diesen gegenüber der Umgebung abzudichten. Dabei ist das Dichtelement 3 auf der zu dem Behälter liegenden Seite der Außenkappe 2 angeordnet.

Die Außenkappe 2 besteht aus einem harten Material, vorzugsweise Kunststoff, das im folgenden als Kunststoff-Hartkomponente bezeichnet wird. Im Prinzip ist es jedoch auch möglich, die Außenkappe 2 bei Bedarf aus Metal herzustellen. Das Dichtelement 3 ist hingegen aus einem weichelastischen, vorzugsweise gasdichten Material gefertigt. Dazu eignen sich besonders thermoplastische Kunststoffe, die hier auch als Kunststoff-Weichkomponente bezeichnet werden.

In dem Ausführungsbeispiel ist die Außenkappe 2 aus Polypropylen gefertigt, wohingegen das Dichtelement 3 aus Gummi besteht.

Die Außenkappe 2 umfaßt zunächst eine Stirnwand 4 an einem axialen Ende, die von einer Seitenschürze 5 umgeben wird. Die Seitenschürze 5 ist dabei als im wesentlichen zylindrische, bei einer gußtechnischen Fertigung zu der Stirnwand 4 hin leicht konische Hülse ausgebildet, die rückseitig an die Stirnwand 4 anschließt und in dem dargestellten Ausführungsbeispiel im wesentlichen rotationssymmetrisch um eine Längsmittelachse der Verschlußvorrichtung 1 angeordnet ist. Zur Verbesserung der Griffigkeit sind an der Außenwand 6 der Seitenschürze 5 Riffelungen 7, beispielsweise in Form von Längsnuten, vorgesehen.

Die Außenkappe 2 ist an ihrem axialen bzw. äußeren, stirnseitigen Ende nach innen umgestülpt. Die Einstülpung 8 erstreckt sich von der Stirnwand 4 in Form einer Vertiefung in die Außenkappe 2 hinein, wobei die Außenwand 9 der Einstülpung 8 sich zu einem Bodenabschnitt 10 der Vertiefung hin leicht konisch verjüngt. An dem Bodenabschnitt 10 ist mittig eine weitere, nach außen gerichtete Umstülpung vorgesehen, die an dem Boden 10 einen im wesentlichen zylindrischen Vorsprung 11 bildet. Der Vorsprung 11 ist mit einem Durchgang 12 versehen, dessen Mündungsöffnung 13 innerhalb der Vertiefung außerhalb eines Angriffsbereichs eines Fingers von dem Stirnende der Außenkappe 2 zurückgezogen angeordnet ist.

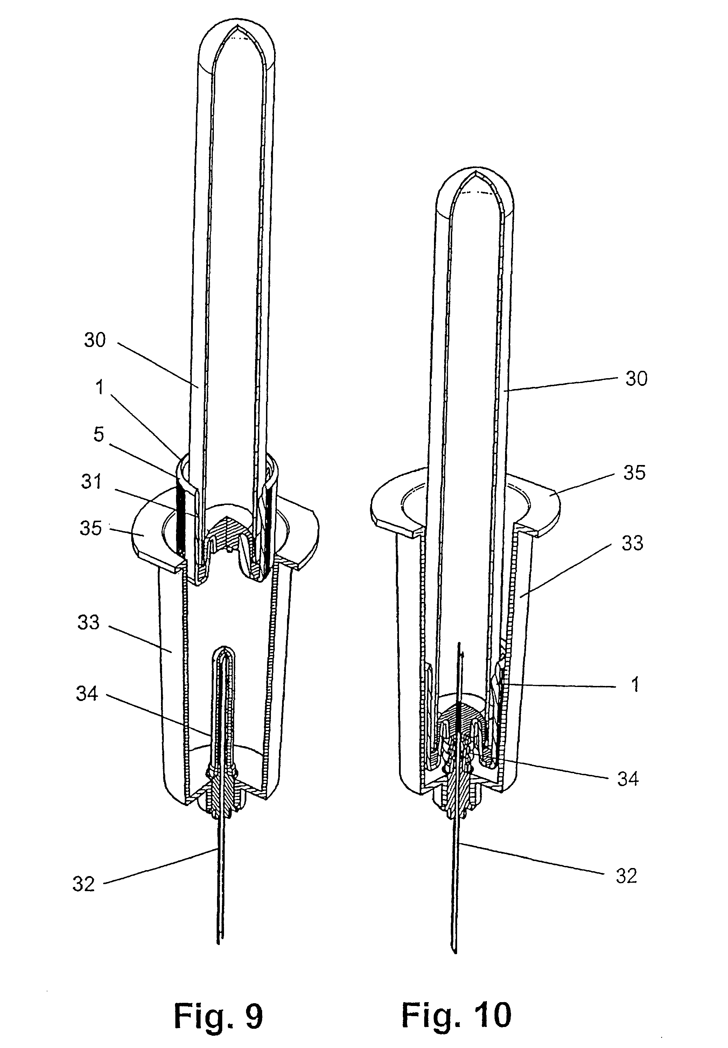

Aus Figur 2 ist zu erkennen, daß der Durchgang 12 vollständig mit Material der Kunststoff-Weichkomponente ausgefüllt ist. Bei der Entnahme einer Blutprobe wird durch das in dem Durchgang 12 befindliche Weichmaterial mit einer Kanüle hindurchgestochen, wie dies in Figur 10 anschaulich dargestellt ist. Wird bei einer Blutentnahme das Blutprobenröhrchen 30 mit der Verschlußvorrichtung 1 von der Kanüle 32 abgezogen, so kann, wie eingangs bereits erläutert, von der Kanüle ein Tropfen abgestreift werden, der in der Vertiefung der Außenkappe 2 außenseitig zunächst an dem Vorsprung 11 hängen bleibt.

Um den Vorsprung 11 ist ein enger, spaltförmiger Ringraum 15 gebildet, in den der Tropfen ablaufen kann. Der Abstand zwischen der Außenwand des Vorsprungs 11 und der Außenwand 9 der Einstülpung 8 ist dabei solchermaßen dimensioniert, daß das 1,5-fache der Wanddicke der Einstülpung 8 an der Außenkappe 2 nicht überschritten wird. Zur Ermöglichung der Aufnahme eines gesamten Tropfens ist der Ringraum 15 ausreichend tief auszubilden. In dem dargestellten Ausführungsbeispiel beträgt die Tiefe des Ringraums 15 in etwa die Hälfte des Abstandes zwischen der Stirnwand 4 dem Bodenabschnitt 10. Durch die spaltartige Ausgestaltung des den Vorsprung 11 umgebenden Raumes 15 wird eine großflächige Verteilung des Bluttropfens vermieden. Vielmehr wird erreicht, daß sich ein solcher mit geringer Oberfläche zur Umgebung in dem Ringraum sammeln kann. Die Ableitung eines solchen Tropfens kann durch eine leicht nach außen gekrümmte Ausbildung der Stirnfläche des Vorsprungs 11 unterstützt werden.

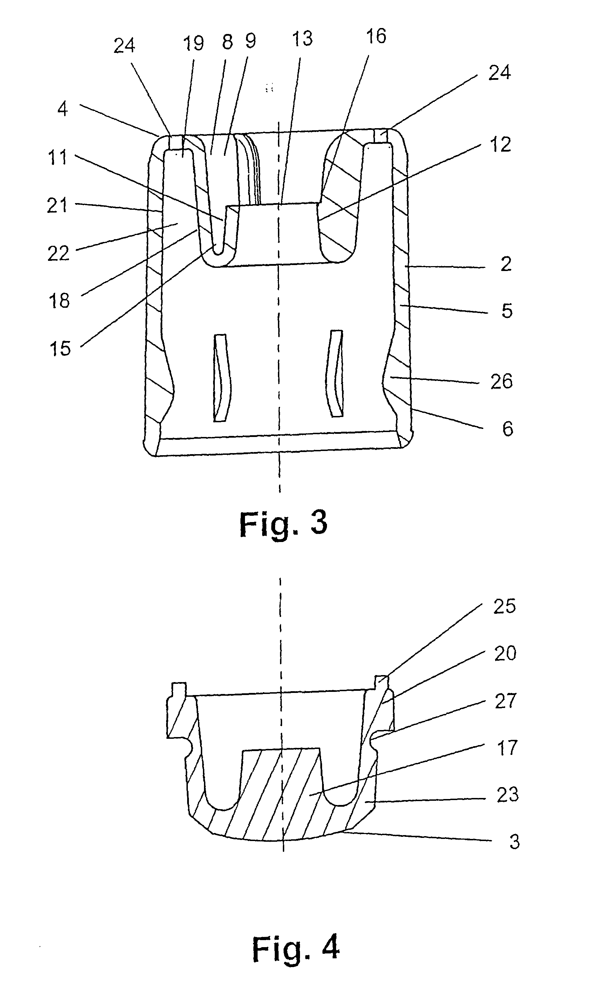

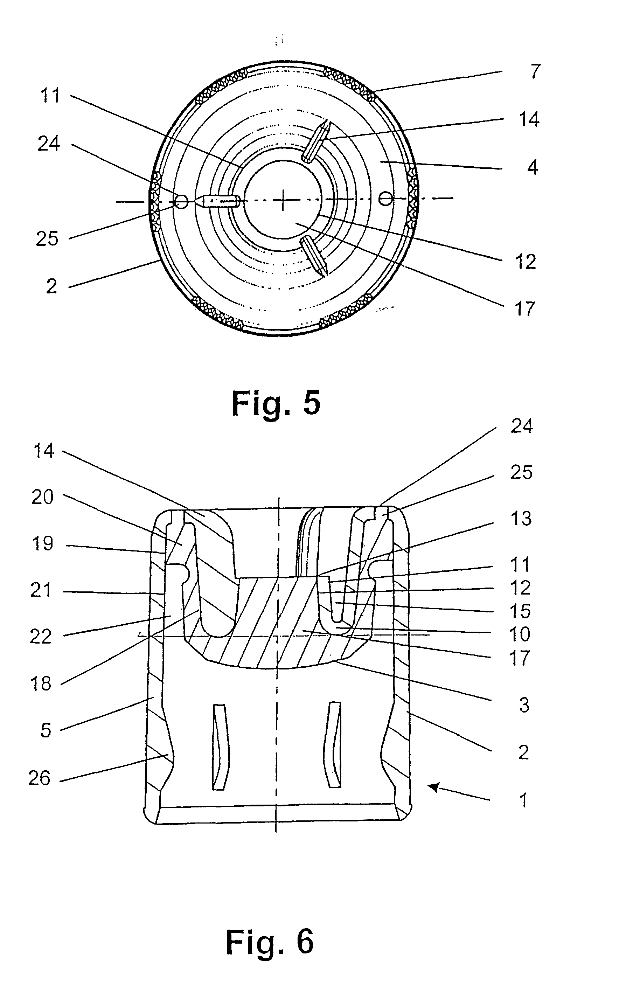

Um das Risiko einer Infektion für die das Blutprobenröhrchen handhabende Person auszuschließen, muß verhindert werden, daß deren Finger mit dem Vorsprung 11 in Berührung kommen können. Hierzu sind an der Außenwand 9 der Einstülpung 8 Rippen 14 vorgesehen, die den freien Durchmesser der Vertiefung bzw. der Einstülpung 8 verringern, so daß auch eine Fingerspitze nicht bis zu dem Vorsprung 11 vordringen kann. Die in dem Ausführungsbeispiel drei gleichbeabstandeten Rippen 14 setzen jeweils an der Stirnwand 4 an und verlaufen bis zu dem Bodenabschnitt 10. Dabei sind, wie insbesondere aus den Figuren 2, 3 und 6 zu erkennen ist, die Rippen 14 auch mit dem Vorsprung 11 verbunden, so daß der Ringraum 15 in hier drei Taschen unterteilt wird. Jede der Taschen ist allein ausreichend dimensioniert, um einen Tropfen in üblicher Größe aufzunehmen. Bei einem einseitigen Ablaufen des Tropfen kann somit eine weitere Verringerung der Oberfläche zur Umgebung erzielt werden. Neben einer Verminderung des freien Durchmessers bewirkten die Rippen 14 weiterhin eine Versteifung der Einstülpung 8 sowie des Vorsprungs 11, die damit dünner ausgebildet werden können, als dies ohne Rippen 14 möglich wäre.

Wie insbesondere Figur 2 entnommen werden kann, füllt das Dichtelement 3 den glattflächigen Durchgang 12 der Außenkappe 2 dicht aus und endet an dem Innenrand 16 der Mündungsöffnung 13, wodurch ein dicker zentraler Dichtpfropfen 17 gebildet wird. Zudem ist der Einstechbereich an dem Vorsprung 11 visuell gut erkennbar. Das Dichtelement 3 erstreckt sich weiterhin entlang der Innenwand 18 der Einstülpung 8 in eine umlaufende Hohlkehle 19, die an einem Stirnende der Außenkappe 2 innenseitig zwischen der Seitenschürze 5 und der Einstülpung 8 gebildet ist. Die Hohlkehle 19 ist mit Material des Dichtelementes 3 ausgefüllt, wozu dieses dort einen Dichtringabschnitt 20 aufweist. Zwischen der Innenwand 21 der Seitenschürze 5 und dem Dichtelement 3 bleibt ein Ringspalt 22 bestehen, der zur dichtenden Aufnahme des hülsenartigen Öffnungsabschnittes 31 des Unterdruck-Probensammelbehälters 30 dient. Ein sicheres Abdichten wird dabei durch die Anlage des hülsenartigen Öffnungsabschnittes 30 gegen die harte Innenwand 21 der Seitenschürze 5 erzielt, wobei der Öffnungsabschnitt jedoch auch gegen den Dichtringabschnitt 20 des Dichtelementes 3 sowie einen Überbrückungsabschnitt 23 zwischen dem Dichtringabschnitt 20 und dem Dichtpfropfen 17 zur Anlage kommt. Die Elastizität des Dichtelementes 3 unterstützt dabei die Andruckkraft des Öffnungsabschnittes gegen die Innenwand 21 der Seitenschürze 5. Durch die Anlage an die Kunststoff-Hartkomponente wird vermieden, daß bei einer unsachgemäßen Aufbringung von Querkräften auf die Verschlußkappe 1 der Unterdruck in dem Blutprobenröhrchen durch eine Verformung des Dichtelementes 3 abgebaut werden kann.

Da das Dichtelement 3 nicht zur Aussteifung der Außenkappe 2 benötigt wird, kann die Materialmenge der Kunststoff-Weichkomponente gering gehalten werden.

Die in dem Ausführungsbeispiel dargestellte Verschlußvorrichtung 1 wird in einem Zweikomponenten-Spritzgießverfahren hergestellt, so daß zwischen der Außenkappe 2 und dem Dichtelement 3 Verbundhaftung besteht. Zur Ausfüllung der Hohlkehle 19 sind im Bereich der Stirnseite 4 der Außenkappe 2 in dem Ausführungsbeispiel zwei Durchgangsöffnungen 24 vorgesehen, durch die die Weichkomponente nach dem Herstellen der Außenkappe 2 eingespritzt werden kann. Die Durchgangsöffnungen 24 sind anschließend ebenfalls mit Material der Weichkomponente ausgefüllt. Durch die entsprechenden Anspritzvorsprünge 25 an dem Dichtelement 3 ergibt sich auch im Bereich der Durchgangsöffnungen 24 an der Stirnseite 4 bei der fertigen Verschlußvorrichtung 1 eine glattflächige Außenform. Überdies wird hierdurch selbst bei einer sehr dünnwandigen Ausbildung des Überbrückungsabschnittes 23 ein zuverlässiges Ausfüllen der Hohlkehle 19 ermöglicht.

Zur Festlegung der Verschlußvorrichtung 1 an dem Unterdruck-Probensammelbehälter sind an einem über den Boden 10 der Vertiefung heruntergezogenen Abschnitt der Seitenschürze 5 an deren Innenwand 21 Rastvorsprünge 26 ausgebildet, die mit gegebenenfalls an dem hülsenartigen Öffnungsabschnitt 31 des Behälters 30 vorgesehenen Ausnehmungen zusammenwirken. Der heruntergezogene Abschnitt dient zugleich als Spritzschutz, wenn die Verschlußvorrichtung 1 in einem Untersuchungslabor von dem Blutprobenröhrchen abgenommen wird. Bei der Aufhebung des Restunterdrucks entstehende Spritzer werden hierdurch abgeschirmt. Überdies kann an dem Überbrückungsabschnitt 23 des Dichtelementes 3 eine Ringnut 27 vorgesehen werden, um einen etwas breiteren Rand, wie er bisweilen bei aus Glas gefertigten Blutprobenröhrchen auftritt, aufzunehmen.

Die Verwendungsweise der Verschlußvorrichtung 1 mit einem Blutprobenröhrchen 30 ist in den Figuren 7 und 8 dargestellt. Bei einer Blutentnahme wird hiernach zunächst eine doppelendige Kanüle 32 mit einem Ende in eine Vene einer Person eingestochen. Alternativ kann die Kanüle 32 auch über eine Verbindungsleitung zu einer weiteren, in eine Person einzustechenden Kanüle leiten. Die Kanüle 32 ist dabei mittig an einem Ende eines hülsenartigen Halters 33 befestigt und an ihrem zweiten Ende mit einer folienartigen Schutzumhüllung 34 umgeben. Der Halter 33 ist an seinem der Kanüle 32 gegenüberliegenden Ende offen und weist dort zusätzlich einen sich im wesentlichen radial erstreckenden Haltesockel 35 auf. Durch das offene Ende wird das Blutprobenröhrchen 30 mit der Verschlußvorrichtung 1 eingeführt und gegen das zweite Ende der Kanüle 32 gedrückt. Dabei wird sowohl die folienartige Schutzumhüllung 34 als auch der Durchgang 12 bzw. der in diesem angeordnete Dichtpfropfen 17 der Verschlußvorrichtung 1 mittig durchstochen. Anschließend erfolgt die Befüllung des Blutprobenröhrchens 30, die durch den in dem Blutprobenröhrchen 30 vorherrschenden Unterdruck unterstützt wird.

Nach der Befüllung wird das Blutprobenröhrchen 30 von der Kanüle 32 abgezogen und in ein Untersuchungslabor gebracht. Gegebenenfalls werden in der zuvor beschriebenen Weise weitere, gleichartige Blutprobenrönrchen 30 befüllt, bis nach der Befüllung des letzten Röhrchens dann die Kanüle 32 aus der Vene herausgezogen wird. Der Dichtpfropfen 17 verschließt dabei automatisch die durch die Kanüle 32 verursachte Öffnung, so daß eine Kontamination der Probe mit Fremdstoffen aus der Umgebung vermieden wird. Durch die oben bereits beschriebene Ausgestaltung der Verschlußvorrichtung 1 wird ein infektionssicherer Transport der Probe in das Untersuchungslabor gewährleistet.

## Patentansprüche

1. Verschlußvorrichtung für einen Unterdruck-Probensammelbehälter, umfassend eine Außenkappe (2) aus einem harten Material, vorzugsweise aus einer Kunststoff-Hartkomponente, mit einer Stirnwand (4) und einer diese umgebenden Seitenschürze (5), die auf einen hülsenartigen Öffnungsabschnitt (31) des Unterdruck-Probensammelbehälters (30) aufsetzbar ist, und ein in der Außenkappe (2) behälterseitig angebrachtes Dichtelement (3) aus einem elastischen Dichtmaterial, vorzugsweise einer Kunststoff-Weichkomponente, wobei die Stirnwand (4) der Außenkappe (2) nach innen und eine Vertiefung bildend eingestülpt ist und der Boden (10) dieser Einstülpung (8) wiederum nach außen gerichtet umgestülpt ist, und diese Umstülpung als ein im wesentlichen hohlzylindrischer Vorsprung (11) ausgebildet ist, der einen glattflächigen Durchgang (12) aufweist, dessen Öffnung (13) in einem Abstand von einem Stirnende der Außenkappe (2) mündet, der außerhalb eines Eingriffsbereichs eines Fingers liegt, und daß zwischen der Außenwand des zylindrischen Vorsprungs (11) und der Innenwand (9) der nach innen gerichteten Einstülpung (8) ein enger, spaltförmiger Ringraum (15) gebildet ist, wobei das Dichtelement (3) den Durchgang (12) ausfüllt und weiterhin die Innenwand (18) der Einstülpung (8) sowie eine Hohlkehle (19), die an dem Stirnende der Außenkappe (2) innenseitig zwischen der Seitenschürze (5) und der Einstülpung (8) gebildet ist, auskleidet, wobei zu der Innenwand (21) der Seitenschürze (5) ein Spalt (22) zur dichtenden Aufnahme des hülsenartigen Öffnungsabschnittes (31) des Unterdruck-Probensammelbehälters (30) ausgebildet ist, **dadurch gekennzeichnet, daß** die Höhe des zylindrischen Vorsprungs (11) über dem Boden (10) der Einstülpung (8) etwa die Hälfte der Tiefe der Einstülpung (8) beträgt und daß die Höhe der Seitenschürze (5) in etwa das Doppelte der Tiefe der Einstülpung (8) beträgt und daß an der Innenwand (9) der nach innen gerichteten Einstülpung (8) in die Vertiefung hineinragende, radiale Versteifungsrippen (14) ausgebildet sind.

2. Verschlußvorrichtung nach Anspruch 1, **dadurch gekennzeichnet, daß** sich die radialen Versteifungsrippen (14) bis in den spaltförmigen Ringraum (15) erstrecken und diesen in spaltförmige Taschen unterteilen.

3. Verschlußvorrichtung nach Anspruch 1, **dadurch gekennzeichnet, daß** die maximale Breite des engen, spaltförmigen Ringraums (15) das 1,5-fache der Wanddicke der Einstülpung (8) and der Außenkappe (2) nicht überschreitet.

4. Verschlußvorrichtung nach einem der Ansprüche 1 bis 3, **dadurch gekennzeichnet, daß** das Dichtelement (3) sich bis zu einem Innenrand (16) der Mündungsöffnung (13) des zylindrischen Vorsprungs (11) erstreckt und in dieser endet.

5. Verschlußvorrichtung nach einem der Ansprüche 1 bis 4, **dadurch gekennzeichnet, daß** das durch die Mündungsöffnung (13) und das Dichtelement (3) gebildete Ende des zylindrischen Vorsprungs (11) nach außen ballig ausgebildet ist.

6. Verschlußvorrichtung nach einem der Ansprüche 1 bis 5, **dadurch gekennzeichnet, daß** an dem Stirnende der Außenkappe (2) im Übergang der Seitenschürze (5) zu der Einstülpung (8) mindestens eine Durchgangsöffnung (24) ausgebildet ist, die zu der Hohlkehle (19) leitet.

7. Verschlußvorrichtung nach einem der Ansprüche 1 bis 6, **dadurch gekennzeichnet, daß** die Verschlußvorrichtung (1) als Zweikomponenten-Kunststoffspritzgußteil ausgebildet ist.

8. Verschlußvorrichtung nach einem der Ansprüche 1 bis 7, **dadurch gekennzeichnet, daß** die Außenkappe (2) aus einem gasundurchlässigen Material, vorzugsweise Polypropylen besteht und das Dichtelement (3) aus einem Elastomer, vorzugsweise Gummi besteht.

9. Unterdruck-Probensammelbehälter, vorzugsweise zur Verwendung bei der Blutabnahme, umfassend einen Behälter (30) mit einem hülsenartigen Öffnungsabschnitt (31), der durch eine Verschlußvorrichtung (1) nach einem der Ansprüche 1 bis 8 abgedichtet ist, wobei in dem Behälter (30) ein Unterdruck herrscht.

## Claims

1. Closing device for a vacuum sample collector, comprising an outer cap (2) made from a hard material, preferably from a plastic hard component, with an front wall (4) and a side skirt (5), which surrounds this front wall and can be placed on a sleeve-like opening section (31) of the vacuum sample collector (30), and a sealing element (3) made from a flexible sealing material, preferably from a plastic soft component, placed in the outer cap (2) on the collector, whereby the front wall (4) of the outer cap (2) is bent in, forming a recess, and the floor (10) of this bent in part (8) is, in its turn, bent up in an outwards direction, and this bent up part is formed essentially as a hollow cylindrical projection (11), which has a smooth surfaced passage (12), the opening (13) of which opens at such a distance from the front end of the outer cap (2), as to be outwith the reach of fingers, and that a narrow, split shaped annulus (15) is formed between the outer wall of the cylindrical projection (11) and the inner wall (9) of the inwards directed bent in part (8), whereby the sealing element (3) fills out the passage (12) and further the inner wall (18) of the bent in part (8), and also lines a groove (19), which is formed at the front end of the outer cap (2) on the inside between the side skirt (5) and the bent in part (8), whereby a split (22) is formed at the inner wall (21) of the side skirt (5), in order to accomodate and seal the sleeve-like opening section (31) of the vacuum sample collector (30), **characterised in that** the height of the cylindrical projection (11) over the floor (10) of the bent in part (8) is about half the depth of the bent in part (8), and **in that** the height of the side skirt (5) is about twice the depth of the bent in part (8) and **in that** stiffening ribs (14) are formed radially at the inner wall (9) of the inwards directed bent in part (8) and reach into the recess.

2. Closing device according to claim 1, **characterised in that** the radial stiffening ribs (14) extend into the split shaped annulus (15) and divide the said annulus into split shaped pockets.

3. Closing device according to claim 1, **characterised in that** the maximum width of the narrow, split shaped annulus (15) does not exceed one and a half times the wall thickness of the bent in part (8) and the outer cap (2).

4. Closing device according to one of claims 1 to 3, **characterised in that** the sealing element (3) extends as far as an inner edge (16) of the outlet opening (13) of the cylindrical projection (11) and terminates into the said outlet opening.

5. Closing device according to one of claims 1 to 4, **characterised in that** end of the cylindrical projection (11), formed by the outlet opening (13) and the sealing element (3), is convex shaped on the outside.

6. Closing device according to one of claims 1 to 5, **characterised in that** at least one passage opening (24) leading to the groove (19) is formed at the front end of the outer cap (2) in the transition of the side skirt (5) to the bent in part (8).

7. Closing device according to one of claims 1 to 6, **characterised in that** the closing device (1) is formed as a dual component plastic injection moulded part.

8. Closing device according to one of claims 1 to 7, **characterised in that** the outer cap (2) is made from a gas impermeable material, preferably polypropylene and the sealing element (3) is made from an elastomer, preferably rubber.

9. Vacuum sample collector, preferably for use in taking blood samples, comprising a collector (30) with a sleeve-like opening section (31), which is sealed by means of a closing device (1) according to one of claims 1 to 8, whereby there is a vacuum in the collector (30).

## Revendications

1. Dispositif de fermeture pour un récipient de collecte d'échantillons sous vide, comprenant un capuchon extérieur (2) constitué d'une matière dure, de préférence d'un composant dur de matière plastique, avec une paroi frontale (4) et un tablier latéral (5) entourant celle-ci, qui peut être placé sur une portion d'ouverture (31) de type douille du récipient de collecte d'échantillons sous vide (30), et un élément d'étanchéité (3) placé côté récipient dans la capuchon extéreur (2), en une matière d'étanchéité élastique, de préférence un composant souple de matière plastique, la paroi frontale (4) du capuchon extérieur (2) étant retournée vers l'intérieur en formant un renfoncement, et le fond (10) de ce retournement (8) est de nouveau retourné dirigé vers l'extérieur, et ce retournement est réalisé en tant que saillie (11) sensiblement cylindrique creuse qui présente un passage (12) de surface lisse dont l'ouverture (13) débouche à une distance d'une extrémité frontale du capuchon extérieur (2), qui se situe à l'extérieur d'une zone d'engagement d'un doigt, et en ce qu'entre la paroi extérieure de la saillie (11) cylindrique et la paroi intérieure (9) du retournement (8) dirigé vers l'intérieur, il est formé un espace annulaire (15) étroit en forme de fente, l'élément d'étanchéité (3) remplissant le passage (12) et recouvrant en outre la paroi intérieure (18) du retournement (8) ainsi qu'une gorge creuse (19) qui est formée à l'extrémité frontale du capuchon extérieur (2), sur le côté intérieur, entre le tablier latéral (5) et le retournement (8), une fente (22) étant réalisée, par rapport à la paroi intérieure (21) du tablier latéral (5), pour recevoir de manière étanche la portion d'ouverture (31) de type douille du récipient de collecte d'échantillons sous vide (30), **caractérisé en ce que** la hauteur de la saillie (11) cylindrique au-dessus du fond (10) du retournement (8) est approximativement égale à la moitié de la profondeur du retournement (8), **en ce que** la hauteur du tablier latéral (5) est approximativement égale au double de la profondeur du retournement (8), et **en ce que** sur la paroi intérieure (9) du retournement (8) dirigé vers l'intérieur, sont réalisées des nervures de renfort (14) radiales pénétrant à l'intérieur du renfoncement.

2. Dispositif de fermeture selon la revendication 1, **caractérisé en ce que** les rainures de renfort (14) radiales s'étendent jusque dans l'espace annulaire (15) en forme de fente et divisent celui-ci en poches en forme de fente.

3. Dispositif de fermeture selon la revendication 1, **caractérisé en ce que** la largeur maximale de l'espace annulaire (15) étroit en forme de fente ne dépasse pas 1,5 fois l'épaisseur de la paroi du retournement (8) sur le capuchon extérieur (2).

4. Dispositif de fermeture selon l'une des revendications 1 à 3, **caractérisé en ce que** l'élément d'étanchéité (3) s'étend jusqu'à un bord intérieur (16) de l'ouverture d'embouchure (13) de la saillie (11) cylindrique et se termine dans cette ouverture.

5. Dispositif de fermeture selon l'une des revendications 1 à 4, **caractérisé en ce que** l'extrémité, formée par l'ouverture d'embouchure (13) et l'élément d'étanchéité (3), de la saillie (11) cylindrique, est bombée vers l'extérieur.

6. Dispositif de fermeture selon l'une des revendications 1 à 5, **caractérisé en ce qu'**à l'extrémité frontale du capuchon extérieur (2), dans la transition entre le tablier latéral (5) et le retournement (8), il est réalisé au moins une ouverture de passage (24) qui mène à la gorge creuse (19).

7. Dispositif de fermeture selon l'une des revendications 1 à 6, **caractérisé en ce que** le dispositif de fermeture (1) est réalisé en tant qu'élément moulé par injection en matière plastique à deux composants.

8. Dispositif de fermeture selon l'une des revendications 1 à 7, **caractérisé en ce que** le capuchon extérieur (2) est constitué d'une matière imperméable aux gaz, de préférence en polypropylène, et l'élément d'étanchéité (3) est réalisé dans un élastomère, de préférence du caoutchouc.

9. Récipient de collecte d'échantillons sous vide, de préférence pour utilisation dans le prélèvement de sang, comprenant un récipient (30) avec une portion d'ouverture (31) de type douille qui est rendue étanche par un dispositif de fermeture (1) selon l'une des revendications 1 à 8, une dépression régnant dans le récipient (30).
